(19) Europäisches Patentamt
European Patent Office
Office européen des brevets

(11) Veröffentlichungsnummer: **0 285 768**
**B1**

# (12) EUROPÄISCHE PATENTSCHRIFT

(45) Veröffentlichungstag der Patentschrift: **24.10.90**

(51) Int. Cl.⁵: **C11D 3/32**, C11D 3/37, C11D 1/52

(21) Anmeldenummer: **88101934.3**

(22) Anmeldetag: **10.02.88**

## (54) Verwendung von N-Polyhydroxyalkylfettsäureamiden als Verdickungsmittel für flüssige wässrige Tensidsysteme.

(30) Priorität: **08.04.87 DE 3711776**

(43) Veröffentlichungstag der Anmeldung: **12.10.88 Patentblatt 88/41**

(45) Bekanntmachung des Hinweises auf die Patenterteilung: **24.10.90 Patentblatt 90/43**

(84) Benannte Vertragsstaaten: **CH DE FR GB LI NL SE**

(56) Entgegenhaltungen:
**EP-A- 0 176 151**
**DE-A- 2 553 399**
**GB-A- 1 339 069**
**GB-A- 2 143 841**

(73) Patentinhaber: **HÜLS AKTIENGESELLSCHAFT, - RSP Patente / PB 15 - Postfach 13 20, D-4370 Marl 1(DE)**

(72) Erfinder: **Kelkenberg, Heike, Dr., August-Schmidt-Strasse 18, D-4390 Gladbeck(DE)**
Erfinder: **Engel, Klaus, Dr., Lange Strasse 28, D-4100 Duisburg 14(DE)**
Erfinder: **Ruback, Wulf, Dr., Spiekeroogstrasse 30, D-4350 Recklinghausen(DE)**

EP 0 285 768 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Produktion flüssiger Produkte auf dem Kosmetik- und Waschmittelsektor nimmt ständig zu. Speziell im Bereich der Körperreinigungsmittel sind es die flüssigen Haarshampoos, Schaumbäder und Duschbäder, die in den letzten Jahren zunehmend an Bedeutung gewonnen haben. Flüssige Geschirrspülmittel und flüssige Feinwaschmittel haben sich ebenfalls einen festen Platz auf dem Markt erobert.

Voraussetzung für eine brauchbare flüssige Tensidrezeptur ist eine gute Lagerbeständigkeit. Die Flüssigkeit darf bei Temperaturschwankungen nicht eintrüben bzw. Absetzungen bilden. Außerdem wird eine gute Hautverträglichkeit verlangt. Das Produkt sollte die Haut möglichst nicht entfetten bzw. sollte nicht zu Hautreizungen führen.

Das flüssige Tensidsystem muß jedoch vor allem auch eine Viskosität haben, die dem jeweiligen Verwendungszweck angepaßt ist und sich möglichst variabel einstellen läßt.

So ist die Viskosität ein entscheidendes Kriterium für die Qualität eines flüssigen Tensidpräparates. Es werden z. B. von einem Duschgel sehr hohe Viskositäten gefordert, während ein Haarshampoo gewöhnlich eine fließfähige Flüssigkeit mit einer relativ niedrigen Viskosität von 1 000 bis 4 000 mPa s darstellt.

Bekannte Verdickungsmittel für flüssige Tensidformulierungen sind unter anderem nichtionogene Fettsäure-polyalkylenglykolester, wie ANTIL (Molgewicht ca. 3 000, Goldschmidt AG), sowie seit vielen Jahren nichtionische Fettsäurealkanolamide (vgl. J. Amer. Oil Chemists' Soc. 35, 548 (1958). Als Fettsäurealkanolamid wird in der Praxis bevorzugt das Kokosfettsäurediethanolamid (SUPERAMID) eingesetzt. Es zeigt gegenüber anderen Fettsäurediethanolamiden die besten Verdickungseigenschaften.

Der Grad der Verdickung hängt stark vom Tensidsystem und vom Elektro lytzusatz ab. So ist es z. B. bekannt, daß sek.-Paraffinsulfonate als Tenside in flüssigen Einstellungen Schwierigkeiten bei der Viskositätseinstellung bereiten. Die bekannten, marktgängigen o. a. Verdikkungsmittel zeigen bei sek.-Paraffinsulfonaten selbst in Gegenwart von Elektrolyten keine ausreichenden Verdickungseffekte. Auch bei Einsatz von Tensid-Gemischen, die Paraffinsulfonat enthalten, z. B. Paraffinsulfonat-Ethersulfat, so wie sie häufig empfohlen werden (vgl. Fette - Seifen - Anstrichmittel 78, 200 (1976), kommt es zu Problemen mit der praxisüblichen Viskositätseinstellung.

Es bestand somit die Aufgabenstellung, Verdicker für flüssige wäßrige Tensidsysteme zu finden, welche den unterschiedlichen Anforderungen der Praxis in Hinblick auf Tensid, Elektrolyt und Einsatzzweck genügen und insbesondere bei Anwesenheit von sek.-Paraffinsulfonaten als Tensiden eine gute Wirkung erbringen.

Diese Aufgabenstellung wurde gelöst durch Verwendung von N-Polyhydroxyalkylfettsäureamiden der allgemeinen Formel

$$\text{I} \quad R_1 - \underset{\underset{O}{\|}}{C} - \underset{\underset{X}{|}}{N} - R_2,$$

in der

$R_1$ ein gegebenenfalls verzweigter Alkylrest mit 1 bis 17, vorzugsweise 7 bis 17 Kohlenstoffatomen,
$R_2$ Wasserstoff, ein, gegebenenfalls verzweigter, gegebenenfalls ungesättigter, Alkylrest mit 1 bis 18, vorzugsweise 1 bis 6 Kohlenstoffatomen oder ein Rest

$$-\!\!\left( CH_2 - \underset{\underset{R_3}{|}}{CH} - O \right)_n H$$

mit n = 0 oder 1 bis 5 und $R_3$ gleich Wasserstoff oder $-CH_3$ sein kann und
X für einen Polyhydroxyalkylrest mit 4 bis 7 Kohlenstoffatomen steht, der gegebenenfalls mit einem Mono-, Di- oder Oligosaccharidrest glykosidisch verknüpft ist,
als Verdickungsmittel für flüssige wäßrige Tensidsysteme.

X stellt vorzugsweise einen 1-Desoxisorbitylrest dar.

Die erfindungsgemäß einzusetzenden N-Polyhydroxyalkylfettsäureamide sollen in einer Menge von 0,5 bis 30 Gewichtsprozent, vorzugsweise 0,5 bis 10 Gewichtsprozent, bezogen auf das gesamte flüssige Tensidsystem, vorhanden sein.

Sie können gegebenenfalls in Kombination mit bekannten Verdickungsmitteln angewendet werden.

In der allgemeinen Formel I kann sich beispielsweise der Alkylrest $R_1$ von folgenden Carbonsäuren ableiten: Caprylsäure, Caprinsäure, Laurinsäure, Myristinsäure, Palmitinsäure, Stearinsäure.

In der Formel I kann $R_2$ beispielsweise Methyl, Ethyl, n-Propyl, Isopropyl, n-Butyl, Isobutyl, n-Pentyl, Isopentyl, n-Hexyl, Isohexyl, n-Octyl, 2-Ethylhexyl, n-Nonyl, Isononyl, n-Decyl, Isodecyl, Dodecyl, Tridecyl, Tetradecyl, Hexadecyl oder Stearyl, 2-Hydroxyethyl, 2-Hydroxypropyl bedeuten.

X kann für folgende Polyhydroxyalkylreste stehen: 1-Desoxierythrityl-, 1-Desoxiarabityl-, 1-Desoxixylityl-, 1-Desoxisorbityl-, 2-Desoxisorbit-2-yl-, 1-Desoximannityl-, 2-Desoximannit-2-yl-, 1-Desoxigalaktityl-, 1-Desoxi-4-glucosido-sorbityl-, 1-Desoxi-4-galaktosido-sorbityl-, 2-Desoxi-4-glucosido-sorbit-2-yl-, 2-Desoxi-4-glucosido-mannit-2-yl-, 1-Desoxi-4-maltoglucosido-sorbityl-, 1-Desoxi-4- oligoglucosido-sorbityl-, 1-Desoxi-4-polyglucosido-sorbityl-. Vorzugsweise steht X für einen 1-Desoxisorbitylrest.

Es kommen somit als erfindungsgemäß zu verwendende Verdickungsmittel beispielsweise folgende Verbindungen infrage: N-Methyl-Kokosfettsäureglucamid, Stearinsäurelactamid, N-Methyl-Ölsäuremaltamid, N-(2-Hydroxyethyl)-Laurinsäurexylamid, N-Ethyl-Myristinsäure-galaktamid, N-Lauryl-N-(4-Oligoglucosido-sorbityl)-Caprinsäureamid, N-Propyl-N-(2-desoxi-sorbit-2-yl)-decansäureamid.

In den flüssigen wäßrigen Tensidsystemen können z. B. folgende Tenside anwesend sein: anionische Tenside, wie beispielsweise Alkylarylsulfonate, insbesondere Alkylbenzolsulfonate, Olefinsulfonate, sek.-Paraffinsulfonate, Sulfobernsteinsäurehalbestersalze, Fettalkoholethersulfate; nichtionische Tenside, wie z. B. Fettalkoholpolyglykolether, Alkylphenolpolyglykolether, Fettsäurepolyglykolester, Polypropylenoxid-Polyethylenoxid-Mischpolymere.

Ein wesentlicher Vorteil der erfindungsgemäß einzusetzenden Verbindungsklasse gegenüber den gängigen Fettsäurediethanolamiden ist ihre hohe Anpassungsfähigkeit an die jeweiligen Bedürfnisse des Tensidsystems durch die Variationsmöglichkeiten der 3 Substituenten $R_1$, $R_2$ und X.

Während im üblicherweise eingesetzten Fettsäurediethanolamid nur die Alkylkette der Fettsäure variabel ist, läßt sich in der erfindungsgemäß vorgeschlagenen Stoffklasse neben der Fettsäurealkylkette $R_1$, der hydrophobierende Substituent am Stickstoff $R_2$ sowie der hydrophilierende Kohlenhydratrest X variieren. Hieraus lassen sich maßgeschneiderte Verdickungsmittel für flüssige Tensidsysteme herstellen.

Die Herstellung von chemischen Verbindungen der Formel I erfolgt bekanntermaßen durch Umsetzung von Fettsäuren bzw. Fettsäureestern mit gegebenenfalls N-substituiertem Polyhydroxyalkylamin in der Schmelze, gegebenenfalls in Gegenwart von alkalischen Katalysatoren.

Die Polyhydroxyalkylamine werden nach dem allgemein bekannten Verfahren der reduktiven Aminierung von Zuckerderivaten mit flüssigem Ammoniak oder auch anderen Alkylaminen, wie Methylamin, Ethylamin, Octylamin, Laurylamin, Kokosfettamin, Stearylamin, sowie den Alkanolaminen Ethanolamin und Isopropanolamin hergestellt, wobei als Beispiele der Zuckerderivate zu nennen sind: Erythrose, Arabinose, Glucose, Galaktose, Mannose, Fructose, Xylose, Maltose, Lactose, Saccharose, Cellobiose, Maltotriose, Maltodextrine, sowie sonstige Stärkeabbauprodukte, wie Glucosesirup.

Die nachfolgenden Beispiele sollen den Erfindungsgegenstand näher erläutern.

Herstellung von N-Methyl-Kokosfettsäureglucamid

In einem Rührkolben werden 669 g (3,0 Mol) Kokosfettsäuremethylester sowie 585 g (3,0 Mol) N-Methylglucamin unter Zusatz von 3,3 g Na-Methylat allmählich auf 135 °C erhitzt. Das während der Reaktion gebildete Methanol wird im zunehmenden Vakuum bei 100 bis 15 mbar in eine gekühlte Vorlage kondensiert. Nach Beendigung der Methanolentwicklung wird das Reaktionsgemisch in 1,5 l Isopropanol warm gelöst, filtriert und zur Kristallisation gebracht. Nach Filtration und Trocknung werden 882 g (= 76 % d. Th.) wachsartiges N-Methylkokosfettsäureglucamid erhalten.
Erweichungspunkt: 80 bis 84 °C
Basenzahl: 4 mg KOH/g

Auf analogem Wege werden folgende Fettsäureglucamide hergestellt:

| | Ausbeute % | Erweichungspunkt (°C) | Basenzahl mg KOH/g |
|---|---|---|---|
| N-Methyllaurinsäureglucamid | 76 | 94 - 96 | 6 |
| N-Methylmyristinsäureglucamid | 75 | 98 - 100 | 3 |
| N-Methylpalmitinsäureglucamid | 75 | 103 - 105 | 5 |
| N-Methylstearinsäureglucamid | 84 | 96 - 98 | 6 |

<u>Viskositätsverhalten von Tensidlösungen in Gegenwart von Verdickungsmitteln</u>

Vermessen wird das erfindungsgemäß einzusetzende N-Methylkokosfettsäureglucamid.

Als Vergleich dienen Verdickungsmittel des Standes der Technik, wie Kokosfettsäurediethanolamid (MARLAMID DF 1218 der Hüls AG) und das Handelsprodukt ANTIL 141 von Goldschmidt (Polyoxiethylen-propylenglykoldioleat).

Die Viskositätsmessungen erfolgen nach gleichem Verfahren:
Meßgerät: Rotovisco RV 12 mit Rechnersteuerung, Fa. Haake
Meßsystem: MV DIN
Schergefälle: 0 bis $50^{-1}$
Temperatur: 25 °C

Tensid-Konzentration:
10 % Gemisch - 3 Teile Fettalkoholethersulfat MARLINAT 24/28, Hüls AG
1 Teil Paraffinsulfonat MARLON PS 60, Hüls AG

Das erfindungsgemäß einzusetzende N-Methylkokosfettsäureglucamid hat folgende Formel:

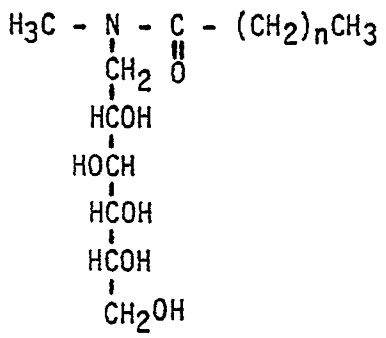

n = 6 bis 16 (Kokos)

Es zeigt in einem Mischsystem Ethersulfat-Paraffinsulfonat deutlich bessere Verdickungseffekte als Kokosfettsäurediethanolamid (vgl. Figur 1).

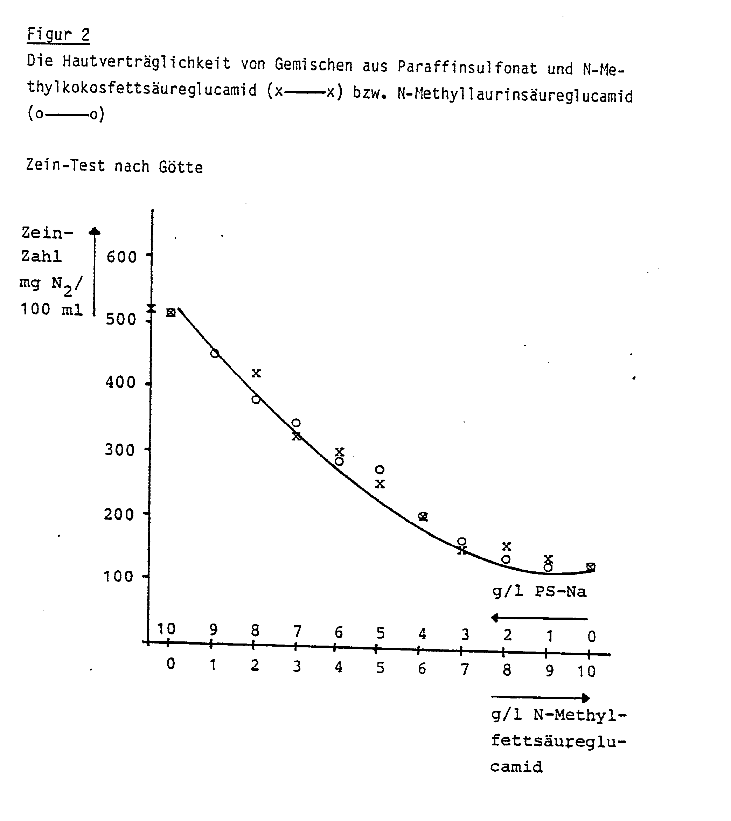

In einer Haarshampoo-Formulierung mit dem besonders gut hautverträglichen Glucamin-Salz der Paraffinsulfonsäure als Basis-Tensid zeigt das N-Methyl-Kokosfettsäureglucamid einen überraschenden synergistischen Verdickungseffekt in Kombination mit dem bekannten Verdicker ANTIL 141 (Goldschmidt AG), der als solcher allein oder auch in Kombination mit Kokosfettsäurediethanolamid auf Paraffinsulfonat-Salze keine Verdikkungswirkung zeigt (vgl. Tabelle I). Formulierungen mit Fettsäureglucamiden sind lagerstabil. Die Hautverträglichkeit von Paraffinsulfonaten wird durch Zusatz von Fettsäureglucamiden erheblich verbessert (vgl. Figur 2).

Tabelle 1

Haarshampoo-Formulierungen auf der Tensidbasis Paraffinsulfonsäure-Na-Salz (PS-Na) bzw. dem hautverträglichen Paraffinsulfonsäure-Glucamin-Salz (PS-Glucamin) in Kombination mit Verdicker-Gemsichen

| Formulierung | I | II | III | |
|---|---|---|---|---|
| Paraffinsulfonsäure-Glucamin-Salz | 13,5 | 13,5 | 13,5 | % |
| MARLAMID (Kokosfettsäurediethanolamid) | | 3,0 | | % |
| N-Methylkokosfettsäureglucamid | | | 3,0 | % |
| ANTIL (Polyoxiethylen-propylenglykoldioleat) | 2,56 | 2,56 | 2,56 | % |
| Viskosität | 142 | 317 | 1 590 | mPa s |

| Formulierung | Ia | IIa | IIIa | |
|---|---|---|---|---|
| Paraffinsulfonsäure-Na-Salz | 13,5 | 13,5 | 13,5 | % |
| MARLAMID | | 3,0 | | % |
| N-Methylkokosfettsäureglucamid | | | 3,0 | % |
| ANTIL | 2,56 | 2,56 | 2,56 | % |
| Viskosität | 63 | 217 | 622 | mPa s |

Die Ausprüfungen zeigen deutlich die Überlegenheit der erfindungsgemäß einzusetzenden N-Polyhydroxyalkylfettsäureamide gegenüber den Verdikkern des Standes der Technik, und zwar sowohl in der eigentlich verdickenden Wirkung als auch in der Hautverträglichkeit.

**Patentansprüche**

1. Verwendung von N-Polyhydroxyalkylfettsäureamiden der allgemeinen Formel

$$R_1 - \underset{\underset{O}{\|}}{C} - \underset{\underset{X}{|}}{N} - R_2$$

in der

$R_1$ ein gegebenenfalls verzweigter Alkylrest mit 1 bis 17 Kohlenstoffatomen,
$R_2$ Wasserstoff, ein, gegebenenfalls verzweigter, Alkylrest mit 1 bis 18 Kohlenstoffatomen oder ein Rest

$$-(CH_2-\underset{\displaystyle R_3}{\underset{|}{CH}}-O)_nH$$

mit n = 0 oder 1 bis 5 und $R_3$ gleich Wasserstoff oder $-CH_3$ sein kann und
X für einen Polyhydroxyalkylrest mit 4 bis 7 Kohlenstoffatomen steht, der gegebenenfalls mit einem Mono-, Di- oder Oligosaccharidrest glykosidisch verknüpft ist,
als Verdickungsmittel für flüssige wäßrige Tensidsysteme.

2. Verwendung von N-Polyhydroxyalkylfettsäureamiden nach Anspruch 1, dadurch gekennzeichnet, daß X einen 1-Desoxisorbitylrest darstellt.

3. Verwendung von N-Polyhydroxyalkylfettsäureamiden nach Anspruch 1 oder 2 in einer Menge von 0,5 bis 30 Gewichtsprozent, bezogen auf das gesamte flüssige Tensidsystem.

4. Verwendung von N-Polyhydroxyalkylfettsäureamiden nach einem der Ansprüche 1 bis 3 in einem flüssigen wäßrigen Tensidsystem, welches wenigstens ein sek.-Paraffinsulfonat enthält.

**Claims**

1. The use of N-polyhydroxyalkyl fatty acid amides of the general formula

$$R_1-\underset{\displaystyle O}{\underset{\|}{C}}-\underset{\displaystyle X}{\underset{|}{N}}-R_2$$

in which,
$R_1$ represents a branched or unbranched alkyl radical having 1 to 17 carbon atoms,
$R_2$ represents hydrogen, a branched or unbranched alkyl radical having 1 to 18 carbon atoms or a radical

$$-(CH_2-\underset{\displaystyle R_3}{\underset{|}{CH}}-O)_nH$$

where
n = 0 or 1 to 5 and $R_3$ may be hydrogen or $-CH_3$ and
X represents a polyhydroxyalkyl radical having 4 to 7 carbon atoms which, if desired, is linked glycosidically with a mono-, di- or oligo-saccharide radical,
as thickening agents for liquid aqueous surfactant systems.

2. The use of N-polyhydroxyalkyl fatty acid amides according to claim 1, characterized in that X is a 1-desoxysorbityl radical.

3. The use of N-polyhydroxyalkyl fatty acid amides according to claim 1 or 2 in an amount of 0.5 to 30% by weight, relative to the overall liquid surfactant system.

4. The use of N-polyhydroxyalkyl fatty acid amides according to any of claims 1 to 3 in a liquid aqueous surfactant system which contains at least one secparaffinsulfonate.

**Revendications**

1. L'utilisation, en tant qu'épaississant pour des systèmes tensio-actifs aqueux liquides, d'amides d'acides gras N-poly-hydroxy-alcoyliques de la formule générale

$$R_1-\underset{\displaystyle O}{\underset{\|}{C}}-\underset{\displaystyle X}{\underset{|}{N}}-R_2$$

dans laquelle
$R_1$ représente un radical alkyle éventuellement ramifié comportant de 1 à 17 atomes de carbone,
$R_2$ représente de l'hydrogène, un radical alkyle éventuellement ramifié comportant de 1 à 18 atomes de carbone ou un reste de formule

$$-(CH_2-\underset{\displaystyle R_3}{\underset{|}{CH}}-O)_nH$$

où n est égal à zéro ou a une valeur de 1 à 5 et $R_3$ peut représenter de l'hydrogène ou un groupe $-CH_3$ et X est un radical poly-hydroxy-alkyle comportant de 4 à 7 atomes de carbone éventuellement lié de façon glycosidique avec un radical mono- di- ou oligo-saccharide.

2. L'utilisation d'amides d'acides gras N-polyhydroxy-alkyliques selon la revendication 1, caractérisée par le fait que X représente un radical 1-désoxy-sorbityle.

3. L'utilisation d'amides d'acides gras N-polyhydroxy-alkyliques selon la revendication 1 ou 2, dans une quantité de 0,5 à 30% en poids, relativement à la totalité du système tensio-actif liquide.

4. L'utilisation d'amides d'acides gras N-polyhydroxy-alkyliques selon l'une des revendications 1 à 3 dans un système tensio-actif liquide aqueux qui renferme au moins un paraffine-sulfonate secondaire.

Figur 1

Einfluß von N-Methylkokosfettsäureglucamid und Kochsalz

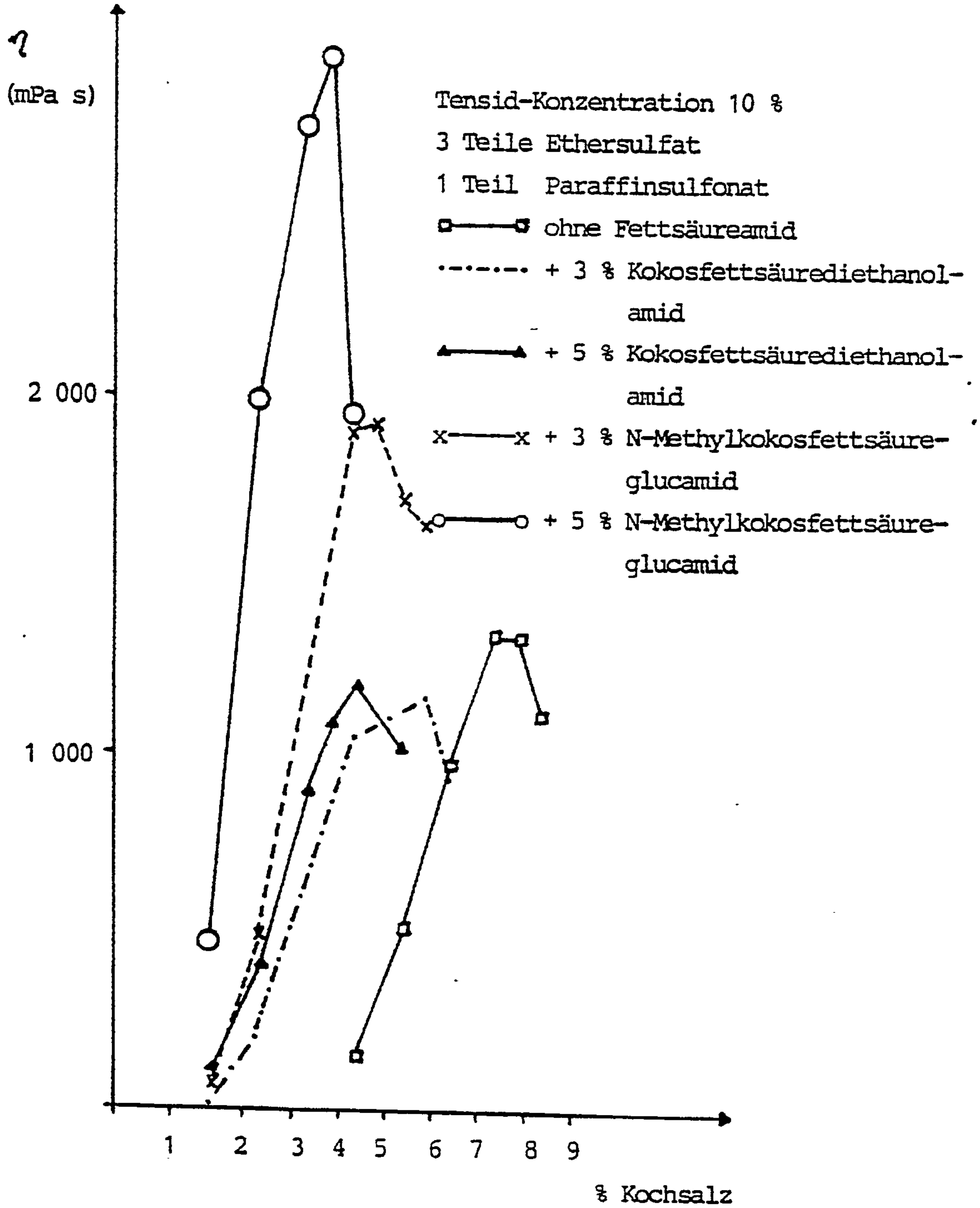

Figur 2

Die Hautverträglichkeit von Gemischen aus Paraffinsulfonat und N-Methylkokosfettsäureglucamid (x——x) bzw. N-Methyllaurinsäureglucamid (o——o)

Zein-Test nach Götte